(19) Europäisches Patentamt / European Patent Office / Office européen des brevets

(11) **EP 3 101 567 A1**

(12) **EUROPEAN PATENT APPLICATION**
published in accordance with Art. 153(4) EPC

(43) Date of publication:
**07.12.2016 Bulletin 2016/49**

(51) Int Cl.:
**G06F 17/50** (2006.01)  **A61B 5/11** (2006.01)

(21) Application number: **15755703.4**

(22) Date of filing: **17.02.2015**

(86) International application number:
**PCT/JP2015/000721**

(87) International publication number:
**WO 2015/129198 (03.09.2015 Gazette 2015/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.02.2014 JP 2014033795**

(71) Applicant: **Hiroshima University**
**Higashi-Hiroshima-shi**
**Hiroshima 739-8511 (JP)**

(72) Inventors:
• **KURITA, Yuichi**
**Higashihiroshima-shi**
**Hiroshima 739-8527 (JP)**

• **TSUJI, Toshio**
**Higashihiroshima-shi**
**Hiroshima 739-8527 (JP)**
• **KONDO, Masaya**
**Higashihiroshima-shi**
**Hiroshima 739-8527 (JP)**
• **KISHISHITA, Yusuke**
**Higashihiroshima-shi**
**Hiroshima 739-8527 (JP)**

(74) Representative: **BRP Renaud & Partner mbB**
**Rechtsanwälte Patentanwälte**
**Steuerberater**
**Königstraße 28**
**70173 Stuttgart (DE)**

(54) **INDUSTRIAL PRODUCT DESIGN SYSTEM, METHOD, PROGRAM, AND COMPUTER-READABLE RECORDING MEDIUM**

(57) An industrial product design system (10) includes: a muscle activity acquisitor (11) that acquires muscle activity required for each action of a given body area when a product user moves the body area to use an industrial product to be designed; a muscle activity normalizer (12) that normalizes the acquired muscle activity; a function operator (13) that calculates, as a design value change rate, mapping of the normalized muscle activity using a given function; and a design value corrector (14) that corrects the design value of the industrial product to be designed with the design value change rate.

FIG.1

# EP 3 101 567 A1

**Description**

TECHNICAL FIELD

The present invention relates to the design of industrial products, and more particularly to an industrial product design technique considering a feeling of kinetic burden by a product user.

BACKGROUND ART

Ergonomics that attempts to utilize physical and physiological features of human beings from an engineering standpoint is being actively applied to the development of human interfaces of various industrial products, among others. Ergonomics-based design is not only easy to use for human beings but also useful in preventing mistakes human beings are likely to make before they occur.

In ergonomics, a human body is represented by various models, and various types of motions of a human body are simulated by a model on a computer. Examples of such models include a finite element model as the most complicated one and a musculoskeletal model as a simple one where the framework, joints, and skeletal muscles of a human body are modeled. For example, as human body kinetic evaluation based on a musculoskeletal model, an evaluation system has been proposed by the present inventors, which, provided with evaluation indices for evaluating skills and sensibilities quantitatively, can automatically calculate a posture close to a posture of human beings (see Patent Document 1, for example).

CITATION LIST

PATENT DOCUMENT

PATENT DOCUMENT 1: Japanese Unexamined Patent Publication No. 2011-141706

SUMMARY OF THE INVENTION

TECHNICAL PROBLEM

When a product user uses an industrial product, doing something such as pressing a button and operating a lever, the user moves his or her body area, thereby having a feeling of kinetic burden. When the feeling of kinetic burden is large, the user feels that the industrial product is hard to use. In reverse, when the feeling of kinetic burden is small, the user feels that the industrial product is easy to use.

The usability of an industrial product felt by the user is sometimes different with the difference of the body size of the user, etc. For example, an industrial product designed with a user having a standard body size in mind may be hard to use for a tall person or a short person. In other words, if the design of an industrial product fails to suit to the body size and muscle force of a user, the user will have a feeling of kinetic burden in a larger amount when using the product, thereby feeling that the product is hard to use. There is therefore a need for such design of an industrial product that will lighten the feeling of kinetic burden by the user. However, since the feeling of kinetic burden is a subjective matter for the user, it is difficult to deal with this sense quantitatively.

In the conventional industrial product design, in many cases, trial subjects have been asked to use trial products produced with various design values, and with fed-back opinions from the trial subjects, the design values have been changed. This method however requires a large amount of labor and time. Therefore, simpler industrial product design is desired.

In consideration of the above problem, it is an objective of the invention to provide an industrial product design technique considering a feeling of kinetic burden by evaluating a feeling of kinetic burden by the product user quantitatively based on objective indices.

SOLUTION TO THE PROBLEM

The industrial product design system according to one aspect of the invention includes: a muscle activity acquisitor that acquires muscle activity required for each action of a given body area when a product user moves the body area to use an industrial product to be designed; a muscle activity normalizer that normalizes the acquired muscle activity; a function operator that calculates, as a design value change rate, mapping of the normalized muscle activity using a given function; and a design value corrector that corrects a design value of the industrial product to be designed with the design value change rate.

The industrial product design method according to another aspect of the invention includes: acquiring muscle activity required for each action of a given body area when a product user moves the body area to use an industrial product to be designed, performed by a muscle activity acquisitor; normalizing the acquired muscle activity, performed by a muscle activity normalizer; calculating, as a design value change rate, mapping of the normalized muscle activity using a given function, performed by a function operator; and correcting a design value of the industrial product to be designed with the design value change rate, performed by a design value corrector.

The industrial product design program, or the computer-readable recording medium, according to yet another aspect of the invention is a computer program, or a medium that stores such a program, that makes a computer function as: a muscle activity acquisition means that acquires muscle activity required for each action of a given body area when a product user moves the body area to use an industrial product to be designed; a muscle activity normalization means that normalizes the acquired muscle activity; a function operation means that calculates, as a design value change rate, mapping of the normalized muscle activity using a given function; and a design value correction means that corrects a design value of the industrial product to be designed with the design value change rate.

The "industrial product" as used herein refers to any of industrially mass-produced ones such as electric home appliances, computers, mobile terminals, and various mechanical products. The industrial product includes, not only the completed one of the product, but also various components thereof. Also, the industrial product design may include arrangement of various types of objects operated by the user, such as buttons displayed on a touch panel screen, etc.

The "given body area" as used herein refers to an arm (superior limb), a finger, a foot (inferior limb), etc.

The "actions" as used herein may include moving a body area to various arrival points, subjecting a body area to reaching movement along various trajectories, moving a body area at various speeds, moving a body area under various load conditions, etc.

According to the above-described industrial product design system, method, and program, a feeling of kinetic burden by the user during use of a product is evaluated by muscle activity, and the design value of the product to be designed is corrected based on the muscle activity.

The muscle activity acquisitor may compute the muscle activity required for each action of the given body area based on a musculoskeletal model of the product user. According to this, the muscle activity can be acquired comparatively easily without cumbersome work of sticking electrodes on a body area of the product user to measure an electromyogram.

The design value may include at least one of a position and color of each part of the industrial product to be designed, a reaction force of the part against operation, a characteristic of vibration of the part during operation, and a contact detection sensitivity of the part during operation.


ADVANTAGES OF THE INVENTION


According to the invention, a feeling of kinetic burden by a product user is evaluated quantitatively based on objective indices, to permit industrial product design considering a feeling of kinetic burden. Thus, various industrial products can be customized to suit to the body size and muscle strength of the product user.


BRIEF DESCRIPTION OF THE DRAWINGS


[FIG. 1] FIG. 1 is a functional block diagram of a main part of an industrial product design system according to an embodiment of the invention.
[FIG. 2] FIG. 2 is a view for explaining example actions of the user during use of a product.
[FIG. 3] FIG. 3 is a graph showing examples of muscle activity required for the actions shown in FIG. 2.
[FIG. 4] FIG. 4 is a graph obtained by normalizing the muscle activity shown in FIG. 3.
[FIG. 5] FIG. 5 is a graph showing a design value change rate obtained from the normalized muscle activity shown in FIG. 4.
[FIG. 6] FIG. 6 is a view showing an example of a keyboard designed by the industrial product design system according to the embodiment.
[FIG. 7] FIG. 7 is a view showing other examples of keyboards designed by the industrial product design system according to the embodiment.
[FIG. 8] FIG. 8 is a view showing an example of product design performed while presenting the user a muscle activity estimated value in real time.
[FIG. 9] FIG. 9 is a view showing another example of product design performed while presenting the user a muscle activity estimated value in real time.

DESCRIPTION OF EMBODIMENTS

An embodiment for carrying out the present invention will be described hereinafter with reference to the accompanying drawings. Note that the invention is not limited to the following embodiment.

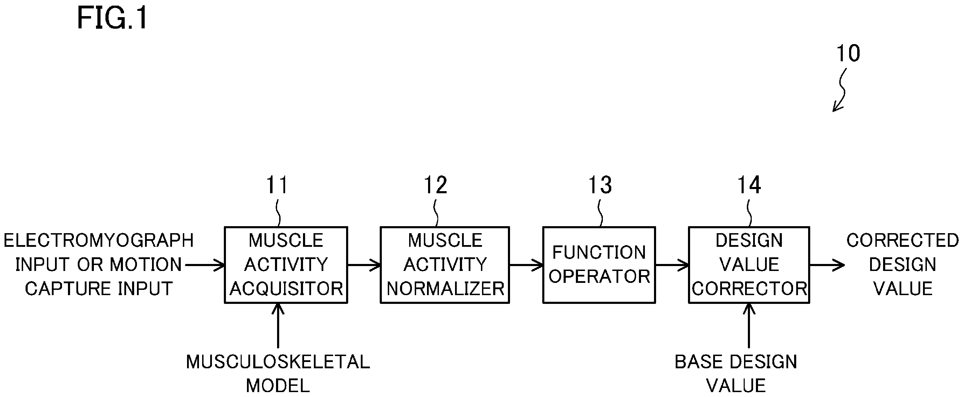

FIG. 1 is a functional block diagram of a main part of an industrial product design system 10 according to an embodiment of the invention. The industrial product to be designed may be any of industrially mass-produced ones such as electric home appliances, computers, mobile terminals, and various mechanical products. The industrial product includes, not only the completed one of the product, but also various components thereof. Also, the industrial product design may include arrangement of various types of objects operated by the user, such as buttons displayed on a touch panel screen, etc.

The industrial product design system 10 according to this embodiment includes a muscle activity acquisitor 11, a muscle activity normalizer 12, a function operator 13, and a design value corrector 14. The industrial product design system 10 can be implemented as dedicated hardware where the above components are comprised of semiconductor integrated circuits, etc. Alternatively, the above components may be described into a computer program, and a general computer such as a PC may be made to execute the computer program, thereby implementing the system 10 on the general computer. Moreover, the industrial product design system 10 can be implemented by a combination of hardware and software.

The muscle activity acquisitor 11 acquires the muscle activity required for each action of a given body area when the product user moves the body area to use the industrial product to be designed. For example, the given body area may be a finger when the product to be designed is a keyboard used for a computer, etc., it may be a foot (inferior limb) when the product to be designed is a pedal of a bicycle, etc., and it may be an arm (superior limb) when the product to be designed is an automobile interior (a steering and seat arrangement, etc.), a touch panel, etc. Examples of the actions include moving the body area to various arrival points, subjecting the body area to reaching movement along various trajectories, moving the body area at various speeds, moving the body area under various load conditions, etc.

The muscle activity acquisitor 11 can acquire the muscle activity directly by measuring an electromyogram using an electromyograph. That is, a plurality of electrodes are stuck on the surface of a given body area of the user, to measure electromyograms during the maximum exertion of the voluntary muscle and during use of the product to be designed. The voluntary contraction strength (%MVC) obtained from the measurement results can be regarded as the muscle activity.

Alternatively, the muscle activity acquisitor 11 can acquire the muscle activity indirectly using a musculoskeletal model. More specifically, the muscle activity acquisitor 11 captures data of the motion and posture of the user who is using the product to be designed from a motion capture (not shown). The muscle activity acquisitor 11 then solves an inverse kinematic problem on the input motion capture data, thereby calculating the angle of each joint of the musculoskeletal model. The muscle activity acquisitor 11 also captures data of external load (external force). The muscle activity acquisitor 11 then solves an inverse kinematic problem from the calculated joint angle and the input external load data, thereby computing the moment of each joint of the musculoskeletal model. The thus-computed joint moment $\tau$ is generally expressed as Equation (1) below.

$$\tau = M(q)\ddot{q} + C(q,\dot{q}) + G(q) - E(q,\dot{q}) \qquad \cdots (1)$$

where M in the first term on the right-hand side represents the inertia force, C in the second term on the right-hand side represents the Coriolis force (centrifugal force), G in the third term on the right-hand side represents the gravity, and E in the fourth term on the right-hand side represents the external force. Also, q represents a generalized coordinate.

The muscle activity can be determined by performing static optimization from the above-computed joint moment. The relationship between the joint moment and the muscle activity is expressed by Equation (2) below.

$$\sum_{m=1}^{n} [\alpha_m f(F_m^0, l_m, v_m)] r_{m,j} = \tau_j \qquad \cdots (2)$$

where n is the number of muscles, $\alpha_m$ is the muscle activity, $F_m^0$ is the isometric maximum muscle force, $l_m$ is the muscle length, $v_m$ is the muscle shortening velocity, f is a function having the isometric maximum muscle force, the muscle length, and the muscle shortening velocity as arguments, $r_j$ is the moment arm, and $\tau_j$ is the joint moment.

The muscle activity $\alpha_m$, indicating the degree of the activity of each muscle, takes on a value between 0 and 1. A value of the muscle activity $\alpha_m$ closer to 1 indicates that the muscle is more activated.

It is said that human beings are unconsciously selecting such a motion that will make the muscle activity minimum. Therefore, at each moment of joint movement, by solving Equation (2) above so that the square sum of the muscle activity be minimum, or specifically, so that the object function J expressed by Equation (3) below be minimum, the muscle activity closer to the actual motion of human beings can be computed.

$$J = \sum_{m=1}^{n} (\alpha_m)^2 \rightarrow \min \qquad \cdots (3)$$

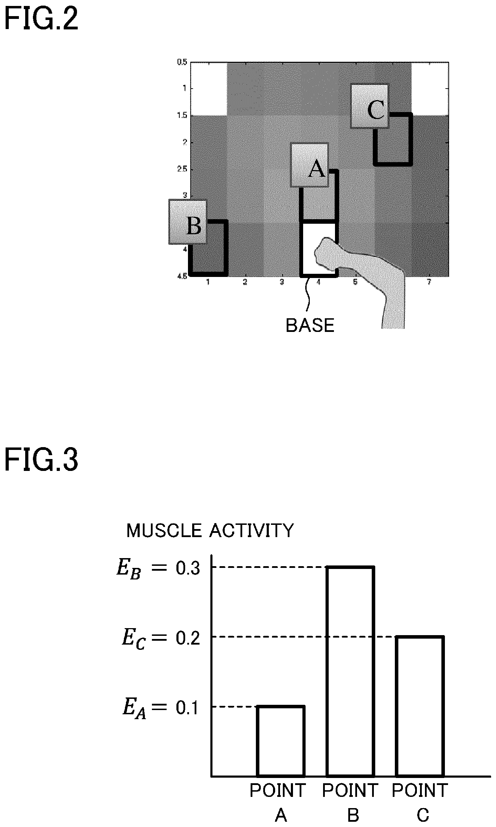

FIG. 2 is a view for explaining example actions of the user during use of a product. Assume, for example, that the product user moves his or her right hand from a base position (BASE) to points A, B, and C (arrival points) during use of the product to be designed. The muscle activity acquisitor 11 acquires the muscle activity of a muscle of the right upper arm required for such actions of the right hand (movements from the base position to points A, B, and C) directly or indirectly in a manner as described above. The muscle activity acquired by the muscle activity acquisitor 11 may be one of a typical muscle of the right upper arm, or an average of muscle activity values of the muscles of the right upper arm.

FIG. 3 is a graph showing examples of the muscle activity required for the actions shown in FIG. 2. For example, the muscle activity $E_A$ required for the action of moving the right hand from the base position to point A (hereinafter referred to as the "point A muscle activity) is 0.1, the muscle activity $E_B$ required for the action of moving the right hand from the base position to point B (hereinafter referred to as the "point B muscle activity) is 0.3, and the muscle activity $E_C$ required for the action of moving the right hand from the base position to point C (hereinafter referred to as the "point C muscle activity) is 0.2.

Referring back to FIG. 1, the muscle activity normalizer 12 normalizes the muscle activity acquired by the muscle activity acquisitor 11. The normalization can be performed according to Equation (4) below, for example.

$$\widehat{E_X} = \frac{E_X - E_{MIN}}{E_{MAX} - E_{MIN}} \qquad \cdots (4)$$

where $E_X$ is the point X muscle activity, $E_{MAX}$ is the maximum muscle activity, $E_{MIN}$ is the minimum muscle activity, and $E_X$ bar is the normalized point X muscle activity.

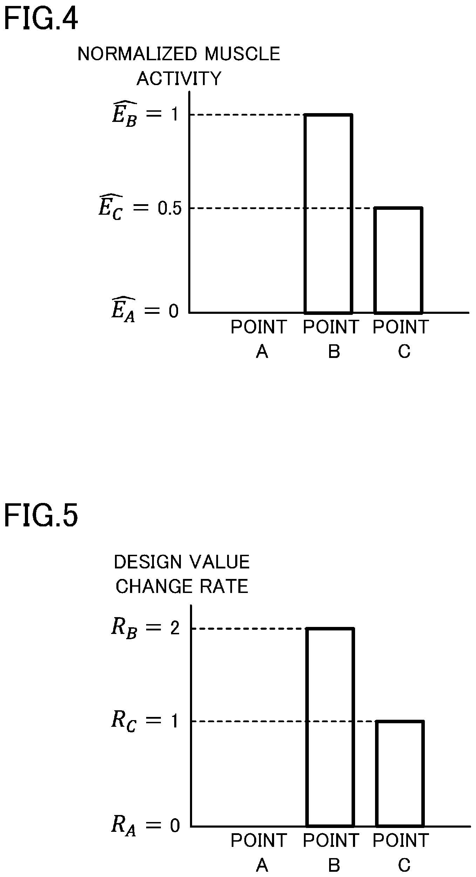

FIG. 4 is a graph obtained by normalizing the muscle activity shown in FIG. 3. In the example in FIG. 3, $E_{MAX}$ is the point B muscle activity $E_B$, and $E_{MIN}$ is the point A muscle activity $E_A$. The normalized point A muscle activity $E_A$ bar (minimum muscle activity) is 0, and the normalized point B muscle activity $E_B$ bar (maximum muscle activity) is 1. The normalized point C muscle activity $E_C$ bar is 0.5 that is a value between 0 and 1.

Referring back to FIG. 1, the function operator 13 calculates, using a given function, mapping of the muscle activity normalized by the muscle activity normalizer 12, and sets the results as design value change rates. As will be described later, the design value change rate is a coefficient for correcting the design value of each part of a product to be designed. For example, the design value change rate $R_X$ at point X of a product to be designed can be calculated according to Equation (5) below.

$$R_X = f\left(\widehat{E_X}\right) \qquad \cdots (5)$$

where f(Z) is the function of Z. As the function f, the linear function (f(Z) = a·Z), the exponential function (f(Z) = a·e$^{b·Z}$), the logarithmic function (f(Z) = a·logZ), etc. can be used. Which function to use can be determined according to the product to be designed.

FIG. 5 is a graph showing the design value change rate obtained from the normalized muscle activity shown in FIG. 4. In the example in FIG. 5, the linear function is used as the function f.

Referring back again to FIG. 1, the design value corrector 14 corrects the design values of the product to be designed with the design value change rates. For example, correction of a design value can be performed according to Equation (6) below.

$$I_X = I_{BASE} + I_s \cdot R_X \qquad \cdots (6)$$

where $I_{BASE}$ is the design base value of the product to be designed (e.g., the design value at the base position shown in FIG. 2), Is is the design change base value of the product to be designed, and $I_X$ is a corrected design value at point X of the product to be designed.

The design value includes at least one of the position and color of each part of the industrial product to be designed, the reaction force of the part against operation, the characteristic of the vibration of the part during the operation, and the contact detection sensitivity. For example, when the product to be designed is a mechanical keyboard, the design value includes the height and position, the color (brightness, chroma, hue, etc.), the magnitude of the reaction force, the hardness of a spring, etc., of each button. Also, when the product to be designed is a touch panel keyboard, the design value may also include the vibration characteristic (frequency, amplitude, vibrating time, etc.) at the pressing of each button, the sensitivity as to how much contact strength is required to detect the contact (contact detection sensitivity), etc.

(Example)

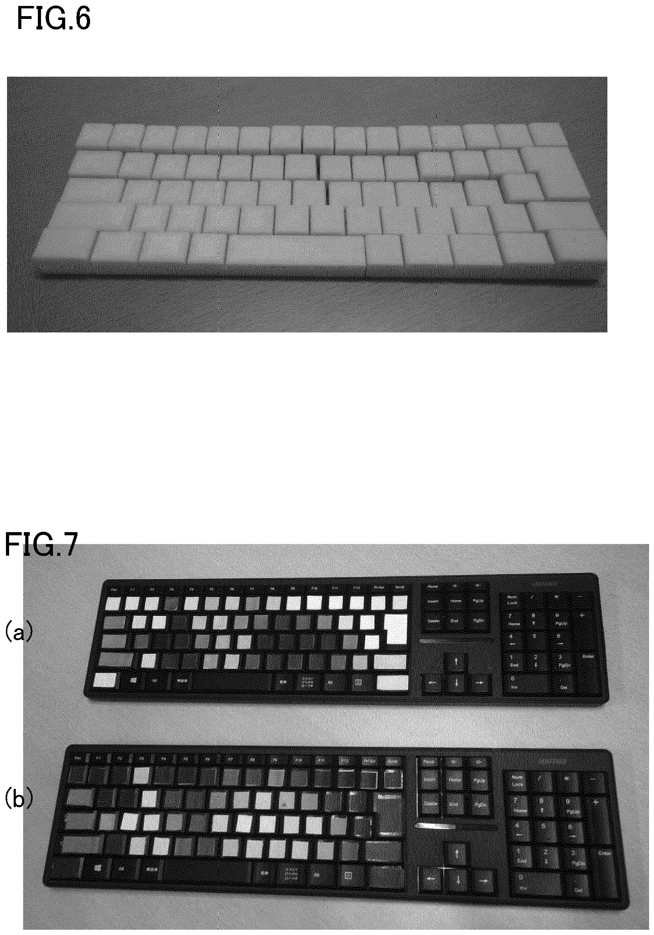

FIG. 6 shows an example of a keyboard designed by the industrial product design system 10. The muscle activity is comparatively large when buttons in the first row, the Enter key, etc., which are located apart from the home position, are pressed. Therefore, these buttons are made a little higher in height than the standard value. With this arrangement, buttons at positions difficult to reach for pressing from the home position become easy to press, whereby the feeling of kinetic burden by the product user can be lightened.

FIG. 7 shows other examples of keyboards designed by the industrial product design system 10. The upper part (a) of FIG. 7 shows an example where, with the standard color of buttons being black, buttons larger in design value change rate are made closer to white. For human beings, white ones appear more rising upward than black ones. Therefore, buttons at positions difficult to reach for pressing from the home position are made white, whereby the feeling of kinetic burden by the product user can be lightened from the visual standpoint. The lower part (b) of FIG. 7 shows an example where, with the standard color of buttons being white, buttons larger in design value change rate are made closer to black. Human beings perceive black ones as being lighter than white ones. Therefore, buttons at positions difficult to reach for pressing from the home position are made black, whereby the feeling of kinetic burden by the product user can be lightened from the visual standpoint.

As described above, according to this embodiment, a feeling of kinetic burden by a product user is evaluated quantitatively based on objective indices, to permit industrial product design considering a feeling of kinetic burden. Thus, various industrial products can be customized to suit to the body size and muscle strength of the product user.

In designing a product using the industrial product design system 10 according to this embodiment, the muscle activity of each body area calculated (estimated) by the muscle activity acquisitor 11 may be presented to the user (in this case, the person who designs the product using the industrial product design system 10) in real time. More specifically, a muscle activity estimated value may be superimposed on an image of the user shot by a camera and displayed on a monitor, using augmented reality (AR) technology. The user can design the product while viewing the muscle activity estimated value-superimposed image.

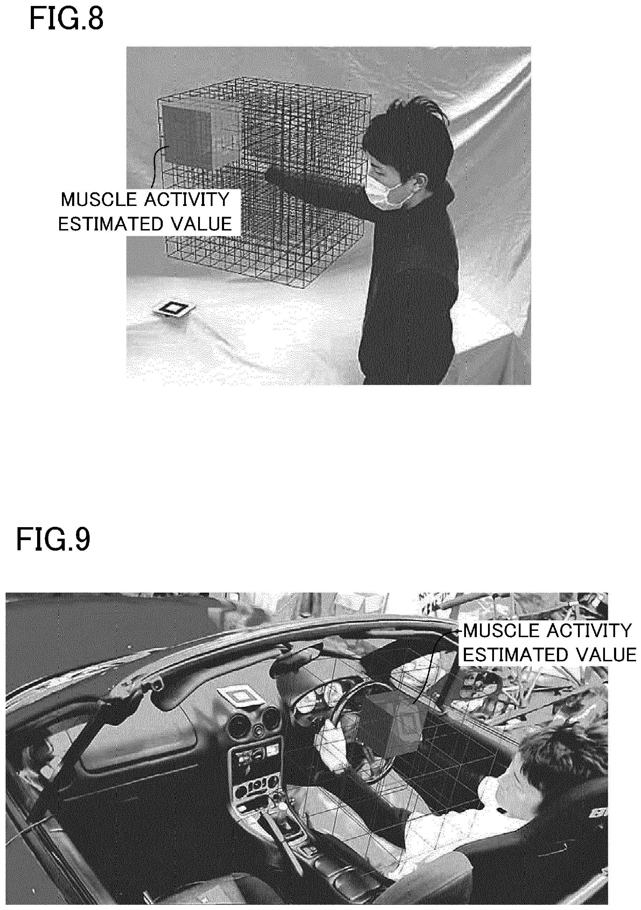

FIG. 8 is a view showing an example of product design performed while presenting the user a muscle activity estimated value in real time. In a case of designing a product, such as a control board and a control panel, which the product user operates with his or her hand by moving his or her upper arm, for example, the calculation result (estimated value) of the muscle activity may be displayed at the position of the hand of the user, as shown in FIG. 8. FIG. 9 is a view showing another example of product design performed while presenting the user a muscle activity estimated value in real time. In a case of determining an optimal seat position of an automobile, for example, the calculation result (estimated value) of the muscle activity may be displayed at the position of a hand of the user who is seated in the automobile holding the steering wheel, as shown in FIG. 9.

In either of the examples in FIGS. 8 and 9, where the muscle activity changes with the motion of the upper arm of the user, the muscle activity estimated value may be presented with change of the color. In this way, by presenting the muscle activity obtained by the muscle activity acquisitor 11 to the user in real time, the industrial product design can be made easier.

DESCRIPTION OF REFERENCE CHARACTERS

10   Industrial Product Design System
11   Muscle Activity Acquisitor
12   Muscle Activity Normalizer
13   Function Operator

14 Design Value Corrector

**Claims**

1. An industrial product design system that designs an industrial product, comprising:

   a muscle activity acquisitor that acquires muscle activity required for each action of a given body area when a product user moves the body area to use an industrial product to be designed;
   a muscle activity normalizer that normalizes the acquired muscle activity;
   a function operator that calculates, as a design value change rate, mapping of the normalized muscle activity using a given function; and
   a design value corrector that corrects a design value of the industrial product to be designed with the design value change rate.

2. The industrial product design system of claim 1, wherein
   the muscle activity acquisitor computes the muscle activity required for each action of the given body area based on a musculoskeletal model of the product user.

3. The industrial product design system of claim 1 or 2, wherein
   the design value includes at least one of a position and color of each part of the industrial product to be designed, a reaction force of the part against operation, a characteristic of vibration of the part during operation, and a contact detection sensitivity of the part during operation.

4. An industrial product design method for designing an industrial product using a computer, comprising:

   acquiring muscle activity required for each action of a given body area when a product user moves the body area to use an industrial product to be designed, performed by a muscle activity acquisitor;
   normalizing the acquired muscle activity, performed by a muscle activity normalizer;
   calculating, as a design value change rate, mapping of the normalized muscle activity using a given function, performed by a function operator; and
   correcting a design value of the industrial product to be designed with the design value change rate, performed by a design value corrector.

5. The industrial product design method of claim 4, wherein
   the muscle activity acquisitor computes the muscle activity required for each action of the given body area based on a musculoskeletal model of the product user.

6. The industrial product design method of claim 4 or 5, wherein
   the design value includes at least one of a position and color of each part of the industrial product to be designed, a reaction force of the part against operation, a characteristic of vibration of the part during operation, and a contact detection sensitivity of the part during operation.

7. An industrial product design program that makes a computer function as a system that designs an industrial product, the program making the computer function as:

   a muscle activity acquisition means that acquires muscle activity required for each action of a given body area when a product user moves the body area to use an industrial product to be designed;
   a muscle activity normalization means that normalizes the acquired muscle activity;
   a function operation means that calculates, as a design value change rate, mapping of the normalized muscle activity using a given function; and
   a design value correction means that corrects a design value of the industrial product to be designed with the design value change rate.

8. The industrial product design program of claim 7, wherein
   the muscle activity acquisition means computes the muscle activity required for each action of the given body area based on a musculoskeletal model of the product user.

9. The industrial product design program of claim 7 or 8, wherein
the design value includes at least one of a position and color of each part of the industrial product to be designed, a reaction force of the part against operation, a characteristic of vibration of the part during operation, and a contact detection sensitivity of the part during operation.

10. A computer-readable recording medium that stores a computer program that makes a computer function as:

   a muscle activity acquisition means that acquires muscle activity required for each action of a given body area when a product user moves the body area to use an industrial product to be designed;
   a muscle activity normalization means that normalizes the acquired muscle activity;
   a function operation means that calculates, as a design value change rate, mapping of the normalized muscle activity using a given function; and
   a design value correction means that corrects a design value of the industrial product to be designed with the design value change rate.

11. The computer-readable recording medium of claim 10, wherein
the muscle activity acquisition means computes the muscle activity required for each action of the given body area based on a musculoskeletal model of the product user.

12. The computer-readable recording medium of claim 10 or 11, wherein
the design value includes at least one of a position and color of each part of the industrial product to be designed, a reaction force of the part against operation, a characteristic of vibration of the part during operation, and a contact detection sensitivity of the part during operation.

EP 3 101 567 A1

# FIG.1

10

ELECTROMYOGRAPH INPUT OR MOTION CAPTURE INPUT →

| 11 MUSCLE ACTIVITY ACQUISITOR | → | 12 MUSCLE ACTIVITY NORMALIZER | → | 13 FUNCTION OPERATOR | → | 14 DESIGN VALUE CORRECTOR | → CORRECTED DESIGN VALUE |

↑ MUSCULOSKELETAL MODEL

↑ BASE DESIGN VALUE

# FIG.2

BASE

# FIG.3

MUSCLE ACTIVITY

$E_B = 0.3$

$E_C = 0.2$

$E_A = 0.1$

POINT   POINT   POINT
A        B        C

EP 3 101 567 A1

## FIG.4

NORMALIZED MUSCLE
ACTIVITY

$\widehat{E_B} = 1$

$\widehat{E_C} = 0.5$

$\widehat{E_A} = 0$

POINT A    POINT B    POINT C

## FIG.5

DESIGN VALUE
CHANGE RATE

$R_B = 2$

$R_C = 1$

$R_A = 0$

POINT A    POINT B    POINT C

# FIG.6

# FIG.7

(a)

(b)

## FIG.8

## FIG.9

## INTERNATIONAL SEARCH REPORT

| International application No. |
| --- |
| PCT/JP2015/000721 |

**A. CLASSIFICATION OF SUBJECT MATTER**
*G06F17/50*(2006.01)i, *A61B5/11*(2006.01)i

According to International Patent Classification (IPC) or to both national classification and IPC

**B. FIELDS SEARCHED**

Minimum documentation searched (classification system followed by classification symbols)
G06F17/50, A61B5/11, A61B5/0488

Documentation searched other than minimum documentation to the extent that such documents are included in the fields searched
| | | | |
| --- | --- | --- | --- |
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2004-178222 A (Matsushita Electric Works, Ltd.), 24 June 2004 (24.06.2004), claims (Family: none) | 1-12 |
| A | JP 2006-160241 A (Toyota Central Research and Development Laboratories, Inc.), 22 June 2006 (22.06.2006), claims; paragraphs [0357] to [0368] (Family: none) | 1-12 |
| A | WO 2013/013170 A1 (SMITH & NEPHEW INC.), 24 January 2013 (24.01.2013), abstract; paragraph [0049] & JP 2014-529314 A & US 2014/0244220 A1 & CA 2842357 A1 & CN 103796609 A | 1-12 |

☐ Further documents are listed in the continuation of Box C.   ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 14 April 2015 (14.04.15) | 21 April 2015 (21.04.15) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (July 2009)

**EP 3 101 567 A1**

REFERENCES CITED IN THE DESCRIPTION

**Patent documents cited in the description**

- JP 2011141706 A